# EUROPEAN PATENT SPECIFICATION

(11) **EP 2 617 440 B1**
(45) Date of publication and mention of the grant of the patent: **11.01.2017**
(21) Application number: 13163820.7
(22) Date of filing: 28.10.2008
(51) Int. Cl.: A61L 27/52, A61L 27/34, A61L 27/54, A61L 27/56, A61L 27/58, A61L 27/44, A61F 2/34, A61F 2/30, A61F 2/38

(54) **Medical implants and methods for delivering biologically active agents**
Medizinische Implantate und Verfahren zur Abgabe biologisch aktiver Stoffe
Implants médicaux et procédés de délivrance d'agents biologiquement actifs

(30) Priority: 29.10.2007 US 983254 P
(43) Date of publication of application: 24.07.2013
(62) Divisional of application: 08846165.2
(73) Proprietor: Zimmer, Inc., Warsaw IN 46580 (US)
(72) Inventor: Fang, Zhibin, Warsaw, IN 46582 (US); Son, Yang W., Granger, IN 46530 (US); Vivanco, Juan, Madison, WI 53726 (US); Zhang, Kai, Woodbury, MN 55125 (US); Levine, Danny L., Mishawaka, IN 46544 (US)
(74) Representative: Mays, Julie

(56) References cited:
- EP-A1- 1 647 242
- EP-A2- 0 395 187
- WO-A1-03/026714

## Description

### BACKGROUND OF THE INVENTION

### 1. Field of the Invention.

The present invention relates to medical implants, such as orthopedic implants of the type used in partial or total joint replacement procedures.

### 2. Description of the Related Art.

Orthopedic implants are used in partial or total joint replacement procedures, such as in hip joint, knee joint, and shoulder joint arthroplasties, for example. Typically, these types of orthopedic implants include a first component associated with a first bone and a second component associated with a second bone, wherein the first and second components articulate with respect to one another. The first and second components may be secured to their respective bones by mechanical interconnection, bone cement, and/or the ingrowth of bone tissue into a porous surface of the implant, referred to as osseointegration.

WO 03/026714 A1 discloses a bone implant for load bearing or non-load bearing application, comprising a porous ceramic matrix, infiltrated with a biodegradable polymer that encapsulates the outer surface of the matrix. A bioactive agent is mixed with the polymer and thus also infiltrates the porous matrix. A controlled release is disclosed, via a control of thickness of polymer layer and possibility to infiltrate within some hollow conduits of the matrix. Furthermore when the porous structure is completely filled with the polymer, toughness is increased and potential for fragmentation at the time of surgical implantation is limited.

### SUMMARY OF THE INVENTION

The present invention relates to medical implants, such as orthopedic implants of the type used in partial or total joint replacement procedures, for example. The implants are as defined in the claims and include a porous substrate, and a bearing portion of a polymeric material, for example, which is at least partially molded within the porous substrate. The bearing portion includes a bearing surface that is exposed to an articulating component of another medical implant, and the porous metal substrate contacts the bone for osseointegration of the bone tissue into the porous substrate to anchor the implant. The porous substrate may include biodegradable carrier materials, in the form of one or more layers, that carry biologically active agents such as antibiotics and bone growth factors, for example. The layers of biodegradable carrier materials may be tailored such that, after implantation of the implants, the biologically active agents are released sequentially and/or over time into the surrounding tissue to reduce the chances of infection and/or to promote osseointegration of the implant, for example.

In one form thereof, the present invention provides an implant. The implant includes a porous substrate, a bearing portion of polymeric material, and at least one biologically active agent. The bearing portion is connected to the porous substrate by infiltration of the polymeric material into at least a portion of the porous substrate, and the bearing portion includes a bearing surface. The at least one biologically active agent is incorporated into another portion of the porous substrate.

In another form thereof, the present invention provides a system for incorporating biologically active agents into an implant. The system includes an implant and a mold. The implant includes a porous substrate and a bearing portion of polymeric material connected to the porous substrate by infiltration of the polymeric material into at least a portion of the porous substrate, the bearing portion including a bearing surface. The mold includes a body that conforms to a shape of the porous substrate and at least one channel configured to direct a fluid including at least one biologically active agent into another portion of the porous substrate of the implant.

In yet another form thereof, the present invention provides a method for incorporating biologically active agents into an implant. The method includes the steps of providing an implant that includes a porous substrate and a bearing portion of polymeric material connected to the porous substrate by infiltration of the polymeric material into at least a portion of the porous substrate, the bearing portion including a bearing surface; and injecting at least one biologically active agent into another portion of the porous substrate.

### BRIEF DESCRIPTION OF THE DRAWINGS

The above-mentioned and other features and advantages of this invention, and the manner of attaining them, will become more apparent and the invention itself will be better understood by reference to the following description of embodiments of the invention taken in conjunction with the accompanying drawings, wherein:
Fig. 1 is a perspective view of an exemplary orthopedic implant, shown as an acetabular cup;
Fig. 2A is a fragmentary sectional view of a portion of the implant of Fig. 1;
Fig. 2B is a schematic representation of Fig. 2A;
Figs. 3A and 3B are depictions of exemplary molding arrangements; and
Figs. 4-7 are further schematic representations of fragmentary sectional views of implants according to alternative embodiments.

Corresponding reference characters indicate corresponding parts throughout the several views. The exemplifications set out herein illustrate embodiments of the invention, and such exemplifications are not to be construed as limiting the scope of the invention any manner.

### DETAILED DESCRIPTION

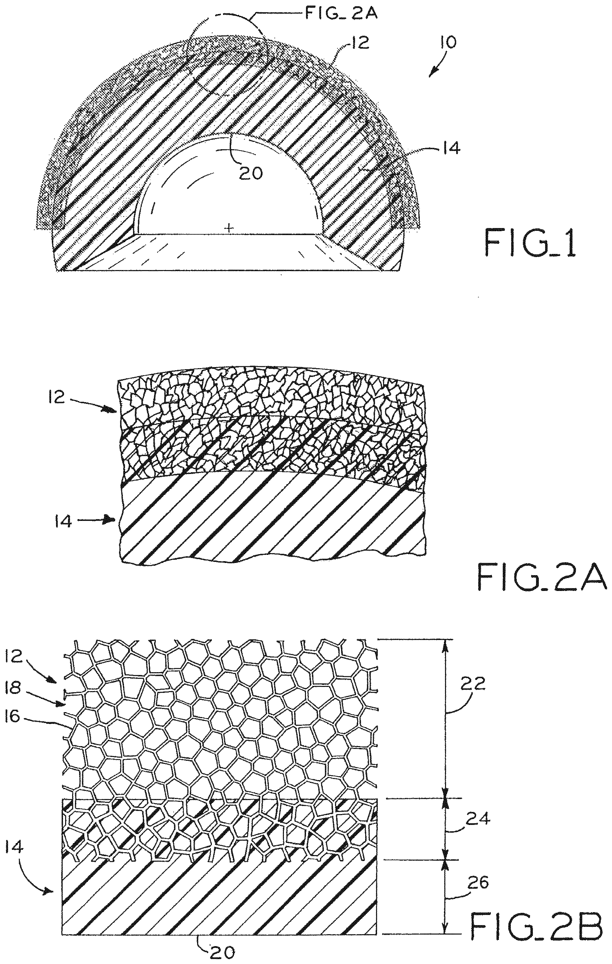

Referring to Fig. 1, an exemplary medical implant is shown in the form of an orthopedic implant and, in particular, an acetabular cup 10 of the type that is implanted within the acetabulum of the pelvis of a patient in a partial or total hip arthroplasty procedure. Acetabular cup 10 generally provides a concave bearing surface that receives the convex articulating head of either the proximal femur itself or of a proximal femoral implant (not shown) that is attached to the femur. Although the present invention is described herein in the form of an orthopedic implant, namely, an acetabular cup, the present invention is generally applicable to any type of medical implant that interfaces with natural tissue, such as bone, when implanted.

Referring to Figs. 1, 2A, and 2B, acetabular cup 10 may be formed as a substantially hemispherical or cup-shaped unitary construct that, as described in detail below, generally includes a porous substrate portion 12 and a bearing portion 14.

Porous substrate portion 12 may be made of a highly porous biomaterial useful as a bone substitute and/or cell and tissue receptive material. An example of such a material is produced using Trabecular Metal™ technology generally available from Zimmer, Inc., of Warsaw, Indiana. Trabecular Metal™ is a trademark of Zimmer Technology, Inc. Such a material may be formed from a reticulated vitreous carbon foam substrate which is infiltrated and coated with a biocompatible metal, such as tantalum, by a chemical vapor deposition ("CVD") process in the manner disclosed in detail in U.S. Patent No. 5,282,861 and in Levine, B.R., et al., "Experimental and Clinical Performance of Porous Tantalum in Orthopedic Surgery", Biomaterials 27 (2006) 4671-4681. In addition to tantalum, other metals such as niobium, or alloys of tantalum and niobium with one another or with other metals may also be used.

Generally, with reference to Fig. 2B, the porous tantalum structure of substrate portion 12 includes a large plurality of ligaments 16 defining open spaces such as voids or channels 18 therebetween, with each ligament 16 generally including a carbon core covered by a thin film of metal such as tantalum, for example. The open spaces between ligaments 16 form a matrix of continuous channels having no dead ends, such that growth of cancellous bone through the porous tantalum structure is uninhibited. The porous tantalum may include up to 75%-85% or more void space therein. Thus, porous tantalum is a lightweight, strong porous structure which is substantially uniform and consistent in composition, and closely resembles the structure of natural cancellous bone, thereby providing a matrix into which cancellous bone may grow to anchor acetabular cup 10 in the surrounding bone of the acetabulum of the pelvis of a patient.

The porous tantalum structure may be made in a variety of densities in order to selectively tailor the structure for particular applications. In particular, as discussed in U.S. Patent No. 5,282,861, the porous tantalum may be fabricated to virtually any desired porosity and pore size, and can thus be matched with the surrounding natural bone in order to provide an improved matrix for bone ingrowth and mineralization.

Bearing portion 14 includes a substantially hemispherical bearing surface 20, and may be formed of a polymeric material such as polyethylene and, in particular, ultra high molecular weight polyethylene (UHMWPE).

Referring to Figs. 2A and 2B, the polymeric material of bearing portion 14 may be molded at least partially within porous substrate 12 to a desired depth to thereby form a unified construct by which the polymeric material of bearing portion 14 is connected to the porous substrate 12 by infiltration of the polymeric material of bearing portion 14 at least partially within the pores or channels 18 of porous substrate 12. In this manner, referring to Fig. 2B, the implant construct generally includes three layers, including a porous layer 22 which will contact and interface with bone tissue when acetabular cup 10 is implanted within a patient, an infiltration layer 24 in which the polymeric material of bearing portion 14 is infiltrated within porous substrate 12, and a bearing layer 26 comprising the polymeric material of bearing portion 14, including bearing surface 20.

As described in detail below, porous layer 22 of the above-described implant construct may include one or more biologically active agents, in the form of one or more layers. After implantation of the implant, the biologically active agent(s) are released or eluted into the surrounding tissue to reduce the chances of infection and/or to promote bony ingrowth, or osseointegration, of bone tissue into porous layer 22 to anchor the implant.

In one embodiment, single or multiple layers of biodegradable carrier materials may be injected into porous layer 22 after bearing portion 14 is molded to porous substrate 12. The biodegradable carrier materials may function as a temporary structural layer to increase the strength of the implant prior to osseointegration, as well as carrier matrix or medium in which the biologically active agent(s) are contained until such time as the implant is implanted. After implantation of the implant, the biodegradable carrier materials will dissolve or resorb into the surrounding tissue, in turn releasing or eluting the biologically active agent(s) into the surrounding tissue.

The biodegradable carrier materials may include biodegradable polymeric materials and/or hydrogels, for example.

Suitable biodegradable polymers that may be used as biodegradable carrier materials include thermoplastic polymers based on poly (ε-caprolactone) (PCL), poly(lactides), or poly(ethylene glycol) (PEG); poly(ortho esters) (POE) and chitosan Poly(DL-lactide), Poly(glycolide), Poly(L-lactide-*co*-glycolide) or Poly(DL-lactide-*co*-glycolide). Natural biopolymers such as chitosan, amphipathic polymers, such as collagen, gelatin and fibrin, and neutral polysaccharides, such as dextran and agarose, may also be used.

Suitable hydrogels that may be used as biodegradable carrier materials include hyaluronic acid, polypropylene fumarate, and Poly(ethylene glycol)-*co*-polylactide, methyl cellulose, and carboxy methyl cellulose. Generally, a hydrogel is a network of polymer chains that are water-soluble but made insoluble through physical and/or chemical crosslinks. These materials are sometimes found as a colloidal gel in which water is the dispersion medium. Hydrogels are generally formed from natural or synthetic polymers. Hydrogels may be classified as "superabsorbent" and may contain over 99% water, by weight. In addition, hydrogels may have the abilty to swell due to water absorption. Hydrogels may also possess a degree of flexibility very similar to natural tissue, due to their significant water content.

Suitable biologically active agents include antibiotics and bone growth factors, for example. Suitable bone growth factors include bone morphogenetic proteins (BMPs) such as BMP-2, -4 and -7, osteoclastogenesis inhibitory factors (OCIF) and geminal bisphosphonates. Suitable antibiotics include Getamicin, Teicoplanin, Aptomycin, Synercid, Linezolid and Tigecycline, for example.

The implant may be designed such that layers that contain antibiotics may be disposed toward the outer regions of the implant that directly interface with, or are positioned proximate, bone tissue, such that the antibiotics are released into surrounding tissues soon after implantation to reduce the possibility of infection and swelling and to promote tissue healing. The biodegradable carrier materials of these layers may be tailored to begin resorbtion, and thereby elution of the biologically active agent(s), within hours or days after implantation, and may require only several hours or a few days, for example, to fully resorb.

Further, the implant may also be designed such that layers that contain bone growth factors may be spaced inwardly from, or beneath, the outer layers of the implant such that, after initial release of antibiotics in the outer layers, bone growth factors are released at a later time to promote full osseointegration of the implant. The biodegradable carrier materials of these layers may be tailored to begin resorbtion, and thereby elution of the biologically active agent(s), after several days or weeks following implantation, and may require several weeks or months, for example, to fully resorb.

In one embodiment, the biodegradable carrier materials and the biologically active agents are mixed and prepared at room temperature or a slightly reduced or elevated temperature, for example, at temperatures that may be as low as 15.56°C, 18.33°C or 21.11°C (60°, 65° or 70°F), or as high as 23.89°C, 26.67°C or 29.44°C (75°, 80° or 85°F). The resulting material will typically be a somewhat viscous liquid that may be injected into porous layer 22 of the implant using a suitable injection device, such as a syringe or an injection molding machine, for example. The material then hardens and solidifies to remain stable until implantation.

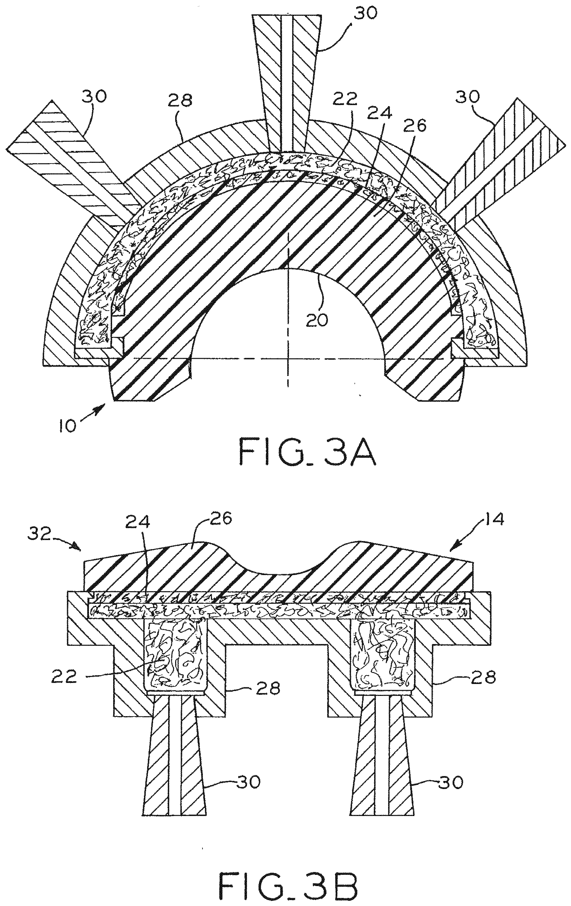

Referring to Figs. 3A and 3B, exemplary depictions of arrangements for direct injection molding of the biodegradable carrier materials into porous layer 22 of implants are shown. In Fig. 3A, porous layer 22 is fitted within a complementary shaped mold body 28, and the biodegradable carrier material is injected through one or more gates or sprues 30 in mold body 28 into porous layer 22. Uniform penetration of the biodegradable carrier material, as well as a desired depth of the biodegradable carrier material, may be achieved by adjusting the pressure, temperature, time, and speed of the injection. A similar molding arrangement is shown in Fig. 3B for another exemplary implant, shown as a tibial implant 32 that includes a porous layer 22 in the form of a tibial base plate and anchor pegs, and a bearing portion 14 against which a distal femoral component (not shown) may articulate.

As discussed below, the implants may include multiple layers of biodegradable carrier materials, which may be achieved in one embodiment by using a solvent removal method. In this method, after a single layer of biodegradable carrier material is injected into porous layer 22, a solvent in which the biodegradable material is soluble or partially soluble is applied to the surface of the layer of biodegradable carrier material to remove a portion of the material, thereby reducing or thinning the layer of biodegradable carrier material to a desired depth. A second layer of biodegradable carrier material may then be injected into porous layer 22 above the first layer. If a third layer of biodegradable carrier material is desired, this process may be repeated as described above with respect to the second layer.

In a similar method, a film of polysulfone thermoplastic, for example, can be used to build multiple layers of biodegradable carrier materials in porous layer 22. In this method, a polysulfone film may be impregnated into porous layer 22 from the surface of porous layer 22 to a desired depth from the surface prior to injecting a biodegradable carrier material in between the film and infiltration layer 24, followed by removal of the film using a suitable solvent such as dichloromethane, for example. Optionally, another layer of biodegradable carrier material may then be injected on top of the first layer of biodegradable carrier material in the space previously occupied by the film.

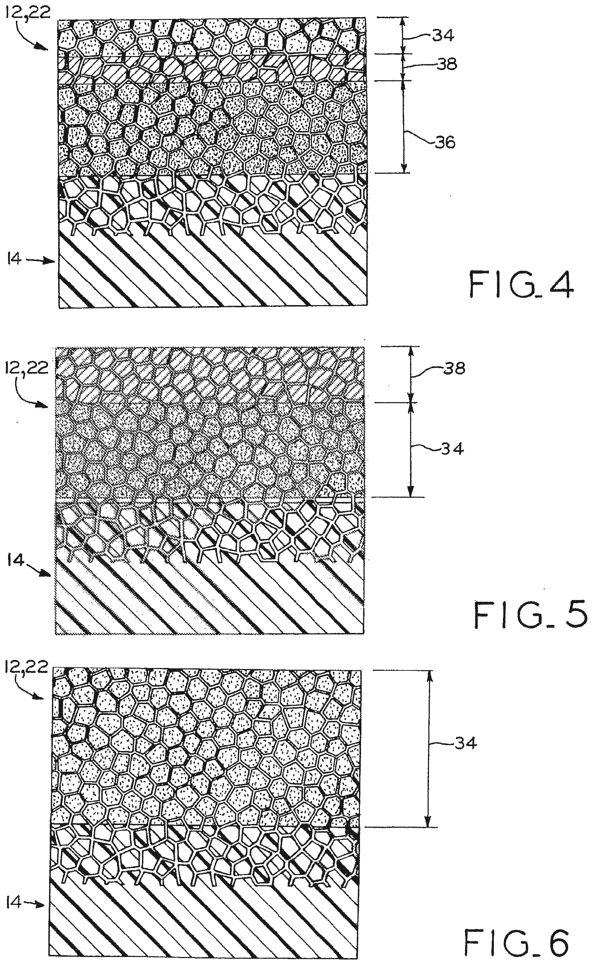

Further exemplary embodiments will now be described with reference to Figs. 4-7. Referring to Fig. 4, in one embodiment, a first layer 34 which, upon implantation of the implant, will be disposed in direct contact with bone, includes a biodegradable carrier material loaded with antibiotics or other pharmaceutical drugs to reduce the possibility of infection and swelling and to promote tissue healing. The resorbtion or elution time of this first layer 28 may be as little as a matter of hours or 1, 2, or 3 days to as long as 1 week, 2 weeks, or 3 weeks, for example.

A second layer 36 is disposed beneath first layer 34 and adjacent the bearing portion 14 of the implant, and may include bone growth factors to promote osseointegration. The resorbtion or elution time of this layer may be as little as 1 week, 2 weeks, or 3 weeks, or as long as 1 month, 2 months, or 3 months, for example.

An optional third layer 38 is disposed between the first and second layers 34 and 36, and may include only a biodegradable carrier material without a biologically active agent. Layer 38 may be tailored to resorb over any of the durations set forth above, and may function as a buffer or barrier layer. In particular, third layer 38 may be tailored to begin resorbtion only after first layer 34 has fully resorbed and eluted its biologically active agent(s), and therefore acts as a buffer layer in the event that full elution of first layer 34 is desired prior to the initiation of the elution of the biologically active agent(s) in second layer 36 to provide a delayed release of the biologically active agent(s) in second layer 36.

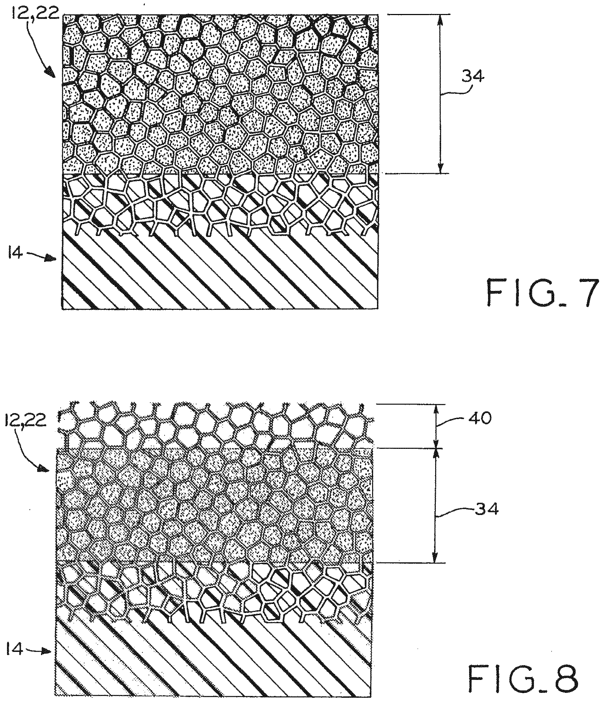

Other configurations are shown in Figs. 5-7. The implant of Fig. 5 includes a barrier layer 38 similar to that of the embodiment of Fig. 4 above, together with a single layer 34 of biodegradable carrier material having one or more biologically active agent(s). The embodiment of Fig. 6 includes only a single, relatively deep or thick layer 34 of biodegradable carrier material having only biologically active agent(s) in the form of antibiotics, for example. The embodiment of Fig. 7 includes only a single, relatively deep or thick layer 34 of biodegradable carrier material having only biologically active agent(s) in the form of bone growth factor(s), for example. The embodiment of Fig. 8 includes an open layer or exposed section 40 of porous portion 12 disposed in contact with the surrounding bone, together with a single, relatively deep or thick layer 34 of biodegradable carrier material having only biologically active agent(s) in the form of bone growth factor(s), for example.

In another embodiment, the initiation of elution, or the speed of elution of the layers of biodegradable carrier material having biologically active agent(s) may be regulated externally of the patient after implantation of the implant using ultrasound, for example, as discussed in co-pending U.S. Provisional Patent Application Serial No. 61/038,852, entitled "Regulation of Medical Device Degradation," filed on March 24, 2008 (Attorney Docket Ref.: ZIM0566). Therefore, the longevity of a porous implant is expected to be increased.

## Claims

1. An orthopaedic implant, comprising:
a porous metallic substrate receptive to bone ingrowth;
a bearing portion including a polyethylene material, connected to the porous metallic substrate through infiltration, and a bearing surface;
a plurality of distinct biodegradable carrier layers which includes a first biodegradable carrier layer and a second biodegradable carrier layer situated within the porous metallic substrate; and
at least one biologically active agent carried by the first biodegradable carrier layer, wherein the first biodegradable carrier layer is situated within the porous metallic substrate between the second biodegradable carrier layer and the polyethylene material infiltrating the porous metallic substrate, wherein the second biodegradable carrier layer comprises a barrier layer for delaying release of the at least one biologically active agent upon implantation of the orthopaedic implant, wherein the barrier layer lacks a biologically active agent.

2. The orthopedic implant of claim 1, wherein the barrier layer is situated within the porous metallic substrate for direct contact with bone upon implantation of the orthopedic implant.

3. The orthopedic implant of claim 1 further comprising a third biodegradable carrier layer situated within the porous metallic substrate and carrying at least one biologically active agent, wherein the second biodegradable carrier layer is situated within the porous metallic substrate between the first biodegradable carrier layer and the third biodegradable carrier layer.

4. The orthopedic implant of claim 3, wherein the first biodegradable carrier layer carries at least a growth factor, and wherein the third biodegradable carrier layer carries at least an antibiotic.

5. The orthopedic implant of claim 1, wherein at least one of the first biodegradable carrier layer and the second biodegradable carrier layer is formed to a desired depth in the porous metallic substrate.

6. The orthopedic implant of claim 1, wherein at least one of the first biodegradable carrier layer and the second biodegradable carrier layer is a hardened layer.

7. The orthopedic implant of claim 1 providing an acetabular cup implant or a proximal tibia implant.

8. A method of forming an orthopedic implant, comprising:
providing an implant comprising:
a porous metallic substrate receptive to bone ingrowth; and
a bearing portion of polyethylene material connected to the porous metallic substrate by infiltration of the polyethylene material into a portion of the porous metallic substrate, the bearing portion including a bearing surface;
injecting a first biodegradable carrier material carrying at least one biologically active agent into the porous metallic substrate to form a first biodegradable carrier layer in the porous metallic substrate; and
injecting a second biodegradable carrier material into the porous metallic substrate to form a second biodegradable carrier layer in the porous metallic substrate, wherein the second biodegradable carrier layer lacks a biologically active agent and comprises a barrier layer for delaying release of the at least one biologically active agent upon implantation, and wherein the first biodegradable carrier layer is situated within the porous metallic substrate between the second biodegradable carrier layer and portions of the polyethylene material infiltrating the porous metallic substrate.

9. The method of claim 8, wherein the barrier layer is situated within the porous metallic substrate for direct contact with bone upon implantation of the orthopedic implant.

10. The method of claim 8 further comprising injecting a third biodegradable carrier material into the porous metallic substrate to form a third biodegradable carrier layer in the porous metallic substrate, wherein the third biodegradable carrier layer carries at least one biologically active agent, and wherein the second biodegradable carrier layer is situated within the porous metallic substrate between the first biodegradable carrier layer and the third biodegradable carrier layer.

11. The method of claim 10, wherein the first biodegradable carrier layer carries at least a growth factor and the third biodegradable carrier layer carries at least an antibiotic.

12. The method of claim 8, wherein at least one of the first biodegradable carrier layer and the second biodegradable carrier layer is formed to a desired depth in the porous metallic substrate.

13. The method of claim 8, wherein at least one of the first biodegradable carrier layer and the second biodegradable carrier layer is a hardened layer.

14. The method of claim 8 forming an acetabular cup implant or a proximal tibia implant.

15. The orthopedic implant of claim 6, or the method of claim 13, wherein the hardened layer comprises a thermoplastic polymer.

## Patentansprüche

1. Orthopädisches Implantat, umfassend:
ein poröses metallisches Substrat, das für ein Einwachsen von Knochen empfänglich ist; einen tragenden Abschnitt enthaltend ein Polyethylenmaterial, verbunden mit dem porösen metallischen Substrat durch Infiltration, und eine tragende Fläche;
eine Vielzahl von verschiedenen biologisch abbaubaren Trägerschichten, was eine erste biologisch abbaubare Trägerschicht und eine zweite biologisch abbaubare Trägerschicht, die sich im porösen metallischen Substrat befindet, enthält; und
mindestens ein biologisch wirksames Mittel getragen von der ersten biologisch abbaubaren Trägerschicht, wobei sich die erste biologisch abbaubare Trägerschicht zwischen dem porösen metallischen Substrat zwischen der zweiten biologisch abbaubaren Trägerschicht und dem Polyethylenmaterial, das das poröse metallische Substrat infiltiert, befindet, wobei die zweite biologisch abbaubare Trägerschicht eine Sperrschicht zum Verzögern einer Freisetzung des mindestens einen biologisch wirksamen Mittels bei Implantation des orthopädischen Implantats umfasst, wobei der Sperrschicht ein biologisch wirksames Mittel fehlt.

2. Orthopädisches Implantat nach Anspruch 1, wobei sich die Sperrschicht im porösen metallischen Substrat für einen direkten Kontakt mit Knochen bei Implantation des orthopädischen Implantats befindet.

3. Orthopädisches Implantat nach Anspruch 1, ferner umfassend eine dritte biologisch abbaubare Trägerschicht, die sich im porösen metallischen Substrat befindet und mindestens ein biologisch wirksames Mittel trägt, wobei sich die zweite biologisch abbaubare Trägerschicht im porösen metallischen Substrat zwischen der ersten biologisch abbaubaren Trägerschicht und der dritten biologisch abbaubaren Trägerschicht befindet.

4. Orthopädisches Implantat nach Anspruch 3, wobei die erste biologisch abbaubare Trägerschicht mindestens einen Wachstumsfaktor trägt, und wobei die dritte biologisch abbaubare Trägerschicht mindestens ein Antibiotikum trägt.

5. Orthopädisches Implantat nach Anspruch 1, wobei mindestens eine der ersten biologisch abbaubaren Trägerschicht und der zweiten biologisch abbaubaren Trägerschicht auf eine gewünschte Tiefe im porösen metallischen Substrat gebildet ist.

6. Orthopädisches Implantat nach Anspruch 1, wobei mindestens eine der ersten biologisch abbaubaren Trägerschicht und der zweiten biologisch abbaubaren Trägerschicht eine gehärtete Schicht ist.

7. Orthopädisches Implantat nach Anspruch 1, das ein Acetabulum-Implantat oder ein proximales Tibia-Implantat bereitstellt.

8. Verfahren zum Bilden eines orthopädischen Implantats,
umfassend: Bereitstellen eines Implantats, umfassend:
ein poröses metallisches Substrat, das für ein Einwachsen von Knochen empfänglich ist; und einen tragenden Abschnitt aus Polyethylenmaterial, verbunden mit dem porösen metallischen Substrat durch Infiltration des Polyethylenmaterials in einen Abschnitt des porösen metallischen Substrats, der tragende Abschnitt enthaltend eine tragende Fläche; Injizieren eines ersten biologisch abbaubaren Trägermaterials, das mindestens ein biologisch wirksames Mittel in das poröse metallische Substrat einträgt, um eine erste biologisch abbaubare Trägerschicht im porösen metallischen Substrat zu bilden; und
Injizieren eines zweiten biologisch abbaubaren Trägermaterials in das poröse metallische Substrat, um eine zweite biologisch abbaubare Trägerschicht im porösen metallischen Substrat zu bilden, wobei der zweiten biologisch abbaubaren Trägerschicht ein biologisch wirksames Mittel fehlt und sie eine Sperrschicht umfasst, um eine Freisetzung des mindestens einen biologisch wirksamen Mittels bei Implantation zu verzögern, und wobei sich die erste biologisch abbaubare Trägerschicht im porösen metallischen Substrat zwischen der zweiten biologisch abbaubaren Trägerschicht und Abschnitten des Polyethylenmaterials, das das poröse metallische Substrat infiltriert, befindet.

9. Verfahren nach Anspruch 8, wobei sich die Sperrschicht im porösen metallischen Substrat für einen direkten Kontakt mit Knochen bei Implantation des orthopädischen Implantats befindet.

10. Verfahren nach Anspruch 8 ferner umfassend ein Injizieren eines dritten biologisch abbaubaren Trägermaterials in das poröse metallische Substrat, um eine dritte biologisch abbaubare Trägerschicht im porösen metallischen Substrat zu bilden, wobei die dritte biologisch abbaubare Trägerschicht mindestens ein biologisch wirksames Mittel trägt, und wobei sich die zweite biologisch abbaubare Trägerschicht im porösen metallischen Substrat zwischen der ersten biologisch abbaubaren Trägerschicht und der dritten biologisch abbaubaren Trägerschicht befindet.

11. Verfahren nach Anspruch 10, wobei die erste biologisch abbaubare Trägerschicht mindestens einen Wachstumsfaktor trägt und die dritte biologisch abbaubare Trägerschicht mindestens ein Antibiotikum trägt.

12. Verfahren nach Anspruch 8, wobei mindestens eine der ersten biologisch abbaubaren Trägerschicht und der zweiten biologisch abbaubaren Trägerschicht auf eine gewünschte Tiefe im porösen metallischen Substrat gebildet ist.

13. Verfahren nach Anspruch 8, wobei mindestens eine der ersten biologisch abbaubaren Trägerschicht und der zweiten biologisch abbaubaren Trägerschicht eine gehärtete Schicht ist.

14. Verfahren nach Anspruch 8, das ein Acetabulum-Implantat oder ein proximales Tibia-Implantat bildet.

15. Orthopädisches Implantat nach Anspruch 6 oder Verfahren nach Anspruch 13, wobei die gehärtete Schicht ein thermoplastisches Polymer umfasst.

## Revendications

1. Implant orthopédique comprenant :
un substrat métallique poreux réceptif à la croissance osseuse ;
une partie d'appui comprenant un matériau en polyéthylène, relié au substrat métallique poreux par infiltration, et une surface d'appui ;
une pluralité de couches supports distinctes biodégradables qui englobe une première couche support biodégradable et une deuxième couche support biodégradable située dans le substrat métallique poreux ; et
au moins un agent biologiquement actif porté par la première couche support biodégradable, où la première couche support biodégradable est située dans le substrat métallique poreux entre la deuxième couche support biodégradable et le matériau de polyéthylène infiltrant le substrat métallique poreux, où la deuxième couche support biodégradable comprend une couche barrière pour retarder la libération dudit au moins un agent biologiquement actif lors de l'implantation de l'implant orthopédique, où la couche barrière manque d'un agent biologiquement actif.

2. Implant orthopédique selon la revendication 1, où la couche barrière est située dans le substrat métallique poreux pour assurer un contact direct avec l'os lors de l'implantation de l'implant orthopédique.

3. Implant orthopédique selon la revendication 1, comprenant en outre une troisième couche support biodégradable située dans le substrat métallique poreux et portant au moins un agent biologiquement actif, la deuxième couche support biodégradable étant située à l'intérieur du substrat métallique poreux entre la première couche support biodégradable et la troisième couche support biodégradable.

4. Implant orthopédique selon la revendication 3, où la première couche support biodégradable porte au moins un facteur de croissance, et où la troisième couche support biodégradable porte au moins un antibiotique.

5. Implant orthopédique selon la revendication 1, où au moins l'une de la première couche support biodégradable et de la deuxième couche support biodégradable est formée à une profondeur désirée dans le substrat métallique poreux.

6. Implant orthopédique selon la revendication 1, où au moins l'une de la première couche support biodégradable et de la deuxième couche support biodégradable est une couche durcie.

7. Implant orthopédique selon la revendication 1, pourvu d'un implant de cotyle ou d'un implant de tibia proximal.

8. Procédé de formation d'un implant orthopédique, comprenant : la fourniture d'un implant comprenant :
un substrat métallique poreux réceptif à la croissance osseuse ; et
une partie d'appui en matériau de polyéthylène reliée au substrat métallique poreux par infiltration du matériau de polyéthylène dans une partie du substrat métallique poreux, la partie d'appui comprenant une surface d'appui ; l'injection d'un premier matériau support biodégradable portant au moins un agent biologiquement actif dans le substrat métallique poreux pour former une première couche support biodégradable dans le substrat métallique poreux ; et
l'injection d'un deuxième matériau support biodégradable dans le substrat métallique poreux pour former une deuxième couche support biodégradable dans le substrat métallique poreux, où la deuxième couche support biodégradable manque d'un agent biologiquement actif et comprend une couche barrière pour retarder la libération dudit au moins un agent biologiquement actif lors de l'implantation, et où la première couche support biodégradable est située à l'intérieur du substrat métallique poreux entre la deuxième couche support biodégradable et des parties du matériau de polyéthylène infiltrant le substrat métallique poreux.

9. Procédé selon la revendication 8, où la couche barrière est située dans le substrat métallique poreux pour assurer un contact direct avec l'os lors de l'implantation de l'implant orthopédique.

10. Procédé selon la revendication 8, comprenant en outre l'injection d'un troisième matériau support biodégradable dans le substrat métallique poreux pour former une troisième couche support biodégradable dans le substrat métallique poreux, où la troisième couche support biodégradable porte au moins un agent biologiquement actif, et où la deuxième couche support biodégradable est située à l'intérieur du substrat métallique poreux entre la première couche support biodégradable et la troisième couche support biodégradable.

11. Procédé selon la revendication 10, où la première couche support biodégradable porte au moins un facteur de croissance et la troisième couche support biodégradable porte au moins un antibiotique.

12. Procédé selon la revendication 8, où au moins l'une de la première couche support biodégradable et de la deuxième couche support biodégradable est formée à une profondeur désirée dans le substrat métallique poreux.

13. Procédé selon la revendication 8, où au moins l'une de la première couche support biodégradable et de la deuxième couche support biodégradable est une couche durcie.

14. Procédé selon la revendication 8, formant un implant de cotyle ou un implant de tibia proximal.

15. Implant orthopédique selon la revendication 6 ou procédé selon la revendication 13, où la couche durcie comprend un polymère thermoplastique.
